# EUROPEAN PATENT APPLICATION

(11) **EP 4 796 063 A1**
(43) Date of publication of application: **26.08.2026**
(21) Application number: 25159253.1
(22) Date of filing: 21.02.2025
(51) Int. Cl.: A61B 18/24, A61B 18/14, A61N 7/02

(54) **SYSTEM FOR TREATING LESION IN VESSEL USING CATHETERS**

(71) Applicant: Koninklijke Philips N.V., 5656 AE Eindhoven (NL)
(72) Inventor: VAN DER HORST, Arjen, Eindhoven (NL); MIEZWA, Megan Ann, Eindhoven (NL); MACCONNELL, Michael Bryan, 5656AG Eindhoven (NL)
(74) Representative: Philips Intellectual Property & Standards

(57) **Abstract**

A catheter system (110) for treating a lesion (112) in a vessel (111) of a subject includes a dilator catheter (120) and a therapy catheter (130) configured for insertion in the vessel. The dilator catheter has a main portion (122) and a tapered portion (123) configured to compress the lesion as the dilator catheter is advanced through the vessel, where the tapered portion increases from a first diameter at a distal end of the tapered portion to a second diameter at a transition between the tapered and main portions. The therapy catheter houses a delivery system (133) for a therapy device (135) configured to remove a portion of the lesion while the lesion is compressed by the dilator catheter.

## Description

### FIELD OF THE INVENTION

The invention relates to the field of medical treatment for intravascular lesions, and more specifically to treating lesions in vessel of a subject using dilator catheters. The invention relates to a dilator catheter for use with a therapy catheter and to a therapy catheter for use with the dilator catheter. The invention also relates to a system comprising the dilator catheter and a therapy catheter, where the system can be for treating a lesion in a vessel of a subject

### BACKGROUND

Venous thromboembolism, including deep vein thrombosis (DVT), is the third most common cardiovascular pathology after coronary artery disease and stroke, and a major contributor to the global disease burden. Lower extremity DVT (LEDVT), in particular, can block the venous lumen and leads to venous congestion, swelling, and lower extremity venous valve function damage, resulting in post-thrombotic syndrome (PTS).

Standard treatments of venous obstructions may include the use of balloons, stents, lytics, and mechanical aspiration. Stents restore flow in PTS cases, but are limited to in treatment location to the iliac veins and leave a permanent implant behind. The implanted stents, and particularly those used to treat DVTs with concurrent inflow disease, are at risk of occluding. Those that do occlude have very limited re-interventional options aside from surgical removal of the stent and may significantly impair the patient's quality of life.

Mechanical thrombectomy treatments involve collection and extraction of the clot, either by mechanical entanglement and transport, or through use of vacuum aspiration. Currently, effective ways of removing clots that have progressed in chronicity to create symptoms of PTS using endovascular methods are lacking. Such post-thrombotic lesions are very tough and wall-adherent, and therefore difficult to remove. When post-thrombotic scarring is located below the common femoral vein, treatment options are limited to percutaneous transluminal angioplasty (PTA), and treatment of these vessels is usually a last attempt to delay the onset of stent occlusion and ultimately re-intervention.

High-pressure balloon venoplasty is currently the only method used to increase lumen area of a vessel by pushing the disease to the side and breaking collagen webs inside the vessel. However, high-pressure balloon venoplasty is not very effective, and the high pressure applied within the lumen may result in vessel damage and re-thrombosis.

### SUMMARY

According to a representative embodiment, a catheter system is provided for treating a lesion in a vessel of a subject. The catheter system includes a dilator catheter and a therapy catheter. The dilator catheter is configured for insertion in the vessel, and has a main portion and a tapered portion configured to compress the lesion as the dilator catheter is advanced through the vessel, where the tapered portion increases from a first diameter at a distal end of the tapered portion to a second diameter at a transition between the tapered portion and the main portion of the dilator catheter, where the second diameter is larger than the first diameter. The therapy catheter is configured for insertion in the vessel, where the therapy catheter houses at least a portion of a delivery system for a therapy device configured to remove a portion of the lesion while the lesion is compressed by the dilator catheter, where the dilator catheter passes through a therapy catheter lumen in the therapy catheter.

According to another representative embodiment, a catheter system is provided for ablating a lesion in a vessel of a subject. The catheter system includes a dilator catheter and a laser catheter configured for insertion in the vessel The dilator catheter has an expandable portion configured to compress the lesion upon inflation, where the expandable portion increases from a first diameter to a second diameter, where the second diameter is larger than the first diameter. Multiple optical fibers pass through the laser catheter and emit laser light, generated by a laser source, from a distal end of the laser catheter while the lesion is compressed by the expandable portion of the dilator catheter, where the dilator catheter passes through a laser catheter lumen of the laser catheter.

According to another representative embodiment, a method is provided for treating a lesion in a vessel of a subject using a catheter system, where the catheter system includes a dilator catheter having a tapered portion that increases from a first diameter at a distal end of the dilator catheter to a second diameter at a transition location proximal to the distal end the dilator catheter, and a therapy catheter including at least a portion of a delivery system for a therapy device configured to remove a portion of the lesion, where the dilator catheter passes through a lumen of the therapy catheter. The method includes advancing the dilator catheter through the vessel past the lesion, where the tapered portion causes the lesion to compress as the dilator catheter moves past the lesion; advancing the therapy catheter through the vessel to the beginning of the lesion; operating the therapy device at a distal end of the therapy catheter to treat the lesion by removing at least a portion of the compressed lesion while advancing the therapy catheter past the lesion; pulling the therapy catheter back to the beginning of the lesion; and determining, using vascular imaging, such as x-ray imaging or vascular ultrasound imaging, for example, whether the treating of the lesion is complete based on the removed portion of the compressed lesion. When it is determined that the treating of the lesion is not complete, the method repeats advancing the therapy catheter through the vessel to the beginning of the lesion and/or rotating the therapy catheter, operating the therapy device at the distal end of the therapy catheter to further treat the lesion by removing an additional at least a portion of the compressed lesion while advancing and/or rotating the therapy catheter past the lesion, pulling the therapy catheter back to the beginning of the lesion, and determining, using the vascular imaging, whether the treating of the lesion is complete based on the additional removed portion of the compressed lesion.

### BRIEF DESCRIPTION OF THE DRAWINGS

The example embodiments are best understood from the following detailed description when read with the accompanying drawing Figures. It is emphasized that the various features are not necessarily drawn to scale. In fact, the dimensions may be arbitrarily increased or decreased for clarity of discussion. Wherever applicable and practical, like reference numerals refer to like elements.
FIG. 1 is a simplified block diagram and partial plan view of a system including a therapy catheter and a dilator catheter for treating a lesion in a vessel of a subject, according to a representative embodiment.
FIG. 2 is a simplified plan view a catheter system including a laser catheter for ablating a lesion in a vessel of a subject, according to a representative embodiment.
FIG. 3 is a simplified plan view a catheter system including a laser catheter for ablating a lesion in a vessel of a subject and markers for accommodating imaging, according to a representative embodiment.
FIG. 4 is a simplified plan view of a catheter system including a laser catheter for ablating a lesion in a vessel of a subject and a steerable dilator catheter, according to a representative embodiment.
FIG. 5 is a simplified plan view of a catheter system for ablating a lesion in a vessel of a subject without a separate laser catheter, according to a representative embodiment.
FIG. 6 is a simplified plan view of a catheter system including a laser catheter for ablating a lesion in a vessel of a subject and a dilator catheter including an expandable device, according to a representative embodiment.
FIG. 7 is a simplified plan view of a catheter system including an expandable laser catheter for ablating a lesion in a vessel of a subject and a dilator catheter including an expandable device, according to a representative embodiment.
FIG. 8A is a simplified plan view of a catheter system including a laser catheter for ablating a lesion in a vessel of a subject and a dilator catheter including a distal IVUS imaging device, according to representative embodiments.
FIG. 8B is a simplified plan view of a catheter system including a laser catheter for ablating a lesion in a vessel of a subject and a dilator catheter including a middle IVUS imaging device, according to representative embodiments.
FIG. 9A is a cross-section of a laser catheter showing a symmetrical arrangement of the corresponding laser catheter lumen and optical fiber openings, according to a representative embodiment.
FIG. 9B is a cross-section of a laser catheter showing an eccentric arrangement of the corresponding laser catheter lumen and optical fiber openings, according to a representative embodiment.
FIG. 10 is a flow diagram of a method of ablating a lesion in a vessel of a subject using a catheter system, according to a representative embodiment.
FIGs. 11A to 11I are simplified plan views a catheter system including a laser catheter for ablating a lesion in a vessel of a subject corresponding to steps in the flow diagram of FIG. 10, according to a representative embodiment.
FIG. 12 is a simplified plan view a catheter system including an RF catheter for ablating a lesion in a vessel of a subject, according to a representative embodiment.
FIG. 13 is a simplified plan view a catheter system including a high intensity ultrasound catheter for ablating a lesion in a vessel of a subject, according to a representative embodiment.
FIG. 14 is a simplified plan view a catheter system including a mechanical catheter for mechanically debulking a lesion in a vessel of a subject, according to a representative embodiment.

### DETAILED DESCRIPTION

In the following detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms "a," "an" and "the" are intended to include both singular and plural forms, unless the context clearly dictates otherwise. Additionally, the terms "comprises," "comprising," and/or similar terms specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

Generally, the various embodiments described herein provide a system and method for breaking a lesion and collagen webs within a vessel in a safe, effective manner using an intravenous dilator catheter and ablation tool, such as a laser. The dilator catheter compresses the lesion and the ablation tool ablates the compressed lesion. The embodiments thus allow for safe opening of the vessel using multiple passes of the ablation tool and the dilator catheter. When the ablation tool is a laser, for example, a laser catheter is provided with optical fiber exposed in a ring-shaped element at the distal tip to provide laser energy ablating the compressed lesion near the dilator catheter. An advantage of laser ablation, in particular, is that no particles generated and no embolic protection devices are needed to prevent pulmonary embolisms.

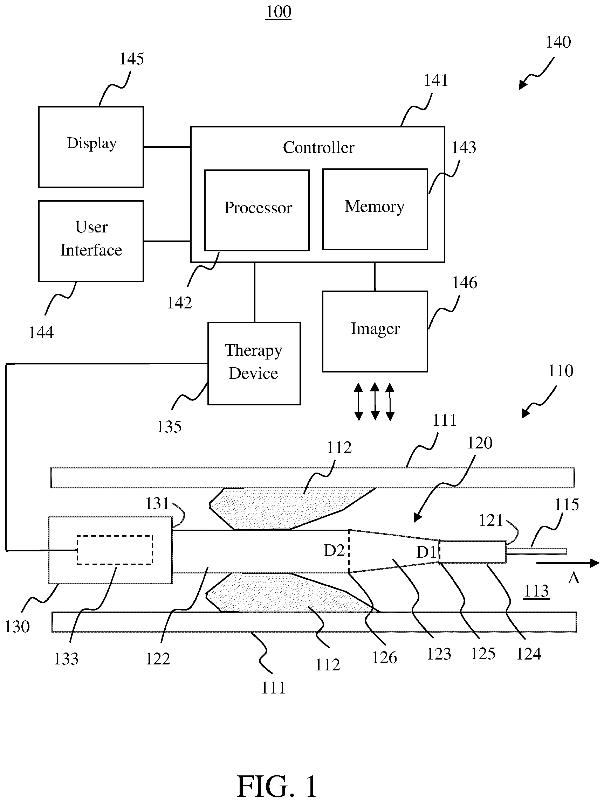

FIG. 1 is a simplified block diagram and plan view of a system for treating a lesion in a vessel of a subject, according to a representative embodiment.

Referring to FIG. 1, the system 100 includes a catheter system 110 configured to be inserted in a vessel 111 of the subject (patient) and a control system 140 configured optionally to control all or part of the operation to remove (debulk) a lesion 112 from the vessel 111. For purposes of illustration, the lesion 112 is shown within a vessel lumen 113 formed by the vessel 111, where the lesion 112 at least partially occludes the subject's blood flow through the vessel 111. The catheter system 110 is shown positioned within the vessel 111 adjacent to the lesion 112 during treatment for removing the lesion 112, as discussed below. The lesion 112 is a mass within the vessel 111, such as a blood clot, thrombus, collagenous lesions/synechia or in-stent thrombosis or post-thrombotic lesion, or atherosclerotic plaque, fibrotic and calcified lesions, or total chronic total occlusion, for example, although other types of lesions may be treated using the catheter system 110 without departing from the scope of the present teachings.

The catheter system 110 includes a dilator catheter 120 and a therapy catheter 130 configured for insertion in the vessel 111, both of which are advanced through the vessel 111 toward the lesion 112. In the depicted embodiment, the catheter system 110 may further include a guide wire 115 configured for initial insertion in the vessel 111. The guide wire 115 may be advanced manually by a user (e.g., physician, clinical technician) of the catheter system 110, or automatically under control of a controller (e.g., controller 101, discussed below), as would be apparent to one skilled in the art. The dilator catheter 120 is inserted in the vessel 111 following the guide wire 115, and the therapy catheter 130 is inserted in the vessel 111 over the dilator catheter 120 also following the guide wire 115. In the depicted embodiment, the guide wire 115 passes through a dilator catheter lumen 121 formed longitudinally in the dilator catheter 120, and the dilator catheter 120 passes through a therapy catheter lumen 131 formed longitudinally in the therapy catheter 130.

The dilator catheter 120 may be moveable (slidable) within the therapy catheter lumen 131 relative to the therapy catheter 130, such that the dilator catheter 120 and the therapy catheter 130 may be advanced through the vessel 111 independently of one another. Alternatively, the dilator catheter 120 may be fixed within the therapy catheter lumen 131 relative to the therapy catheter 130, such that the dilator catheter 120 and the therapy catheter 130 are advanced as a combined unit through the vessel 111. Also, the dilator catheter 120 may include two or more telescoping catheters (not shown), which are separately moveable within the therapy catheter lumen 131. The telescoping catheters are formed such that the respective diameters of the telescoping catchers are smaller in a distal direction toward the distal end of the dilator catheter 120.

The dilator catheter 120 compresses the lesion 112 within the vessel 111, and the therapy catheter 130 treats (e.g., ablates) at least a portion of the lesion 112 while the lesion 112 is in the compressed state. Treatment of the lesion 112 in the compressed state provides more efficient delivery of the treatment modality and results in less debris. To this end, the dilator catheter 120 includes a main portion 122, a tapered portion 123 formed at the distal end of the main portion 122, and an end portion 124 formed at the distal end of the tapered portion 123. In the depicted embodiment, the main portion 122 has a substantially constant diameter along its entire length, where the substantially constant diameter is slightly less than the inner diameter of the therapy catheter lumen 131 to provide a slip fit. The end portion 124 also has a substantially constant diameter along its entire length that is less than the diameter of the main portion 122. The tapered portion 123 increases from a first diameter D1 at a first transition location 125 at its distal end, which coincides with the proximal end of the end portion 124, to a second diameter D2 at a second transition location 126 at its proximal end, which coincides with the distal end the main portion 122. The second diameter D2 is larger than the first diameter D1, where the second diameter D2 is the same as an outer diameter of the main portion 122 and the first diameter D1 is the same as an outer diameter of the end portion 124.

Because the second diameter D2 is larger than the first diameter D1, the tapered portion 123 pushes into the lesion 112 as the dilator catheter 120 is advanced through the vessel 111 in the direction indicated by arrow A. The tapered portion 123 thus compresses the lesion 112. The lesion 112 remains compressed by the main portion 122 as the dilator catheter 120 continues to advance beyond the lesion 112. An outer diameter of the therapy catheter 130 may be about 2mm to about 7mm, the first diameter D1 of the tapered portion 123 may be about 0.4mm to about 1mm, and the second diameter D2 of the tapered portion 123 may be about 1mm to about 6mm, for example.

In the depicted embodiment, the dilator catheter 120 the first transition location 125 is shown at the proximal end of the end portion 124 of the dilator catheter 120. However, it is understood that in various embodiments the dilator catheter 120 does not include the end portion 124, in which case the first transition location 125 may be collocated with the distal tip of the dilator catheter 120 without departing from the scope of the present teachings. Also, in the depicted embodiment, the tapering between the first transition location 125 and the second transition location 126 is linear. However, it is understood that the tapering may increase non-linearly between the first and second transition locations 125 and 126 without departing from the scope of the present teachings.

The therapy catheter 130 houses at least a portion of a delivery system 133 for a therapy device 135, where the delivery system 133 and the therapy device 135 are configured to remove at least a portion of the lesion 112 while the lesion 112 is compressed by the dilator catheter 120, as discussed above. Examples of the therapy catheter 130 for different types of therapy devices and corresponding delivery systems include a laser catheter, a high intensity ultrasound catheter, a radio frequency (RF) catheter, and a mechanical catheter, which are discussed in more detail below. Generally, when the therapy catheter 130 is a laser catheter, the therapy device 135 includes a laser source configured to generate laser light, and the delivery system 133 includes at least one optical fiber passing through the laser catheter for emitting the laser light from a distal end of the laser catheter to ablate at least a portion of the lesion 112. When the therapy catheter 130 is a high intensity ultrasound catheter, the therapy device 135 includes an ultrasound signal source configured to control emission of high intensity ultrasound, and the delivery system 133 includes signal lines passing through the high intensity ultrasound catheter and at least one ultrasound transducer connected to the signal lines for emitting the high intensity ultrasound from the distal end of the high intensity ultrasound catheter to ablate at least a portion of the lesion 112. When the therapy catheter is an RF catheter, the therapy device 135 includes an RF signal source configured to generate RF electric current, and the delivery system 133 includes at least one signal line passing through the RF catheter, and at least one electrode connected to the at least one signal line for emitting the RF electric current from the distal end of the RF catheter to ablate at least a portion of the lesion. When therapy catheter 130 is a mechanical catheter, the therapy device 135 includes a rotating cutter, and the delivery system comprises a motor, a drive train passing through the mechanical catheter and the rotating cutter connected to the drive train at the distal end of the mechanical catheter to mechanically debulk (cut) at least a portion of the lesion.

As mentioned above, the system 100 may further include the control system 140 for implementing and/or managing all or part of the processes described herein with regard to treating the lesion 112 using the catheter system 110. The control system 140 includes a controller 141 configured to interface with the user, control navigation and deployment of the catheter system 110 through the vessel 111, and/or control imaging of the lesion 112 and the catheter system 110 by an imager 146. In the depicted embodiment, the controller 141 includes one or more processors indicated by processor 142 and one or more memories indicated by memory 143. The control system 140 may further include a user interface 144, a display 145, the imager 146, and the therapy device 135, discussed above. The imager 146 may be any compatible vascular imaging modality capable of showing the catheter system 110 and the lesion 112 within the vessel 111 in real time during the treatment procedure. For example, the imager 146 may be an ultrasound imaging device, an x-ray imaging device, or a magnetic resonance (MR) imaging device . When the imager 146 is an ultrasound imaging device, it may include an ultrasound transducer probe that may be manipulated manually by the user, automatically by a robot under control of a robot controller (not shown), or a combination of both. The images may be displayed on the display 145.

The processor 142 communicates with the therapy device 135 through a therapy device interface (not shown) and with the imager 146 through an imaging interface (not shown), as would be apparent to one skilled in the art. The processor 142 is representative of one or more processing devices, and may be implemented by a general purpose computer, a central processing unit (CPU), a digital signal processor (DSP), a graphical processing unit, a computer processor, a microprocessor, a state machine, programmable logic device, field programmable gate arrays (FPGAs), application specific integrated circuits (ASICs), or combinations thereof, using any combination of hardware, software, firmware, hardwired logic circuits, or combinations thereof. Any processor or processing unit herein may include multiple processors, parallel processors, or both. Multiple processors may be included in, or coupled to, a single device or multiple devices. The processor 142 may also refer to a collection of processors within a single computer system or distributed among multiple computer systems, such as in a cloud-based or other multi-site application. Programs have software instructions performed by one or multiple processors that may be within the same computing device or which may be distributed across multiple computing devices.

The memory 143 stores instructions executable by the processor 142 that, when executed, cause the processor 142 to implement all or part of various more processes, as discussed below. The memory 143 may include main memory and/or static memory, where such memories may communicate with each other and the processor 142 via one or more buses. The memory 143 may be implemented by any number, type and combination of random access memory (RAM) and read-only memory (ROM), for example, and may store various types of information, such as software algorithms, artificial intelligence (AI) machine learning models, and computer programs, all of which are executable by the processor 142. The various types of ROM and RAM may include any number, type and combination of computer readable storage media, such as a disk drive, flash memory, an electrically programmable read-only memory (EPROM), an electrically erasable and programmable read only memory (EEPROM), registers, a hard disk, a removable disk, tape, compact disk read only memory (CD-ROM), digital versatile disk (DVD), floppy disk, Blu-ray disk, a universal serial bus (USB) drive, or any other form of storage medium. The memory 143 is a tangible storage medium for storing data and executable software instructions, and is non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The memory 143 may store software instructions and/or computer readable code that enable performance of various functions. The memory 143 may be secure and/or encrypted, or unsecure and/or unencrypted.

The user interface 144 is configured to provide information and data output by the processor 142, the memory 143, the imager 146 and/or the therapy device 135 to the user and/or for receiving information and data input by the user. That is, the user interface 144 enables the user to enter data and to control or manipulate aspects of the processes described herein, and also enables the processor 142 to indicate the effects of the user's input, which may include control or manipulation of the catheter system 110, the therapy device 136, and/or the imager 146. All or a portion of the user interface 144 may be implemented by a graphical user interface (GUI). The user interface 144 may include one or more interface devices, such as a mouse, a keyboard, a trackball, a joystick, a microphone, a video camera, a touchpad, a touchscreen, voice or gesture recognition captured by a microphone or video camera, for example.

The display 145 may be a monitor such as a computer monitor, a television, a liquid crystal display (LCD), an organic light emitting diode (OLED), a flat panel display, a solid-state display, or a cathode ray tube (CRT) display, or an electronic whiteboard, for example. The display 145 includes a screen for viewing images of the catheter system 110 and the lesion 112 within the vessel 111 of the subject during the procedure.

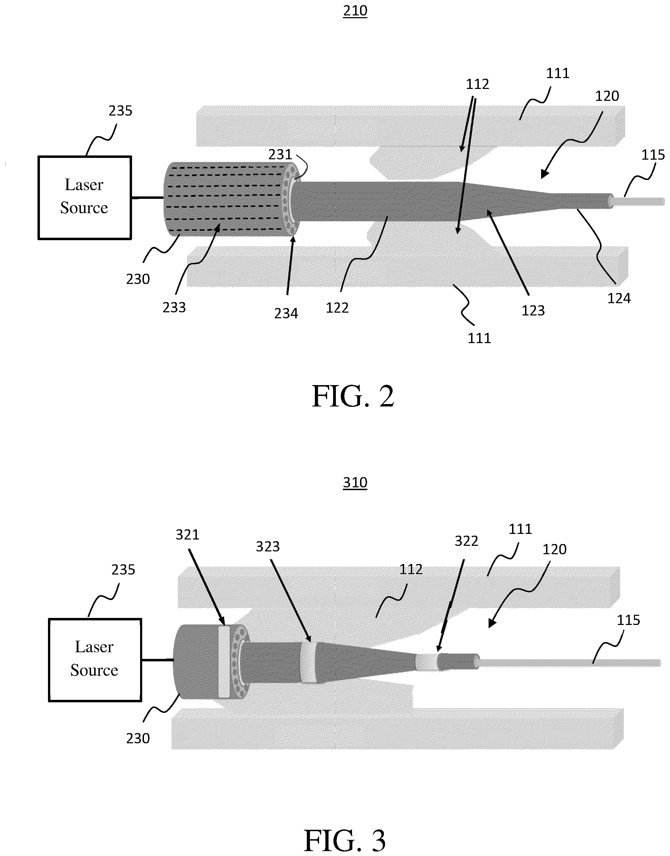

As mentioned above, the catheter system 110 may include different types of therapy catheters for different types of therapy devices and corresponding delivery systems. FIG. 2 is a simplified plan view a catheter system including a laser catheter for ablating a lesion in a vessel of a subject, according to a representative embodiment.

Referring to FIG. 2, catheter system 210 is a laser catheter system shown positioned within the vessel 111 adjacent to the lesion 112 during treatment for removing the lesion 112. The catheter system 210 includes the dilator catheter 120 and a laser catheter 230 configured for insertion in the vessel 111 along the guide wire 115. The dilator catheter 120 passes through a laser catheter lumen 231 formed longitudinally in the laser catheter 230, and may be moveable or fixed therein, as discussed above.

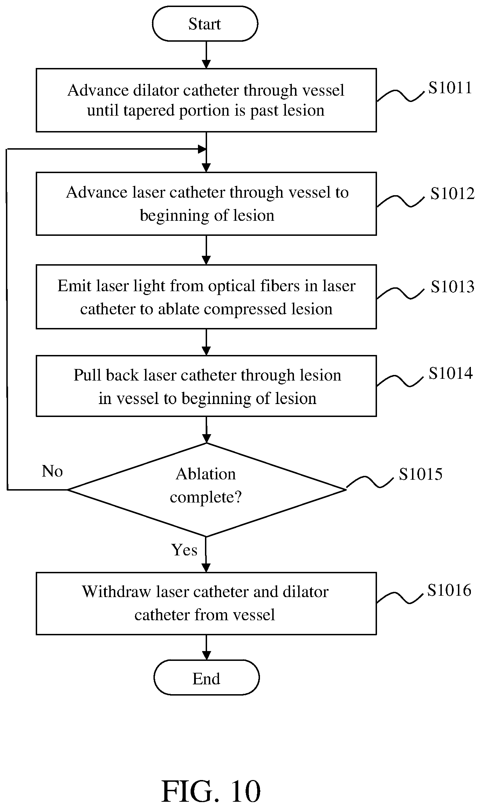

For the laser catheter 230, the therapy device is a laser source 235 configured to generate laser light. The laser source 235 may be a gas laser or a solid-state laser capable of ultraviolet (UV) light generation, for example, having a wavelength in a range of about 126nm to about 360nm, or more specifically in a range of about 300nm to about 360 nm (e.g., a 308nm excimer laser). The delivery system housed in the laser catheter 230 includes one or more end-emitting optical fibers 233 (indicated by representative dashed lines) passing through the laser catheter 230 and emitting the laser light generated by the laser source 235 from corresponding fiber openings 234 at the distal end of the laser catheter 230. The laser light ablates a portion of the lesion 112, while the lesion 112 is compressed by the dilator catheter 120. The process of ablating portions of the lesion 112 may be repeated by advancing and retracting the laser catheter 230 through the lesion 112 until the lesion 112 has been sufficiently eliminated from the vessel 111, which determination may be made by the user. For example, the lesion may be sufficiently eliminated when it has been reduced in size enough to enable substantially unrestricted blood flow, as discussed below with reference to FIG. 10. More particularly, the lesion 112 may be sufficiently eliminated when it has been reduced such that the vessel lumen 113 is equal to or greater than the outer diameter (e.g., second diameter D2) of the dilator catheter 120. The user may then also decide whether to dilate the remainder with another tool, such as a balloon catheter, discussed below. Alternatively, or in addition, the lesion 112 may be sufficiently eliminated and the treatment may be considered complete when the lesion 112 is not recoiling and touching the dilator catheter 120 when the laser catheter 230 is retracted. That is, the ablation is complete when the laser catheter 230 can no longer ablate material of the lesion 112 because the hole in the lesion 112 that has been ablated is the same size or larger than the laser light coverage by the laser catheter 230.

As discussed above, the catheter system 210 may be tracked using the imager 146. In order to enhance tracking ability, the laser catheter 230 and/or the dilator catheter 120 may include markers detectable by the imager 146. FIG. 3 is a simplified plan view of a catheter system including a laser catheter for ablating a lesion in a vessel of a subject and markers for accommodating imaging, according to a representative embodiment.

Referring to FIG. 3, catheter system 310 includes the laser catheter 230 with a first marker 321 arranged near the distal end of the laser catheter 230, and the dilator catheter 120 with a second marker 322 arranged near the first transition location 125 at the distal end portion of the dilator catheter 120, and a third marker 323 arranged near the second transition location 126 of the dilator catheter 120. For example, the first marker 321 may be within 0 to about 5mm of the distal end of the laser catheter 230, the second marker 322 may be within 0 to about 5mm of the first transition location 125, and the third marker 323 may be within 0 to about 5mm of the second transition location 126. The first, second and third markers 321, 322 and 323 are formed of a material visible to the vascular imaging modality of the imager 146. For example, when the imager 146 is an x-ray imaging device, the first, second and third markers 321, 322 and 323 may be formed of a radiopaque material, such as a platinum iridium alloy or a barium sulphate additive to the dilator catheter 120 or (less commonly) gold.

Accordingly, the first marker 321 may be used for guiding the laser catheter 230 through the vessel 111 and positioning the laser catheter 230 immediately adjacent the lesion 112 using the vascular imaging provided by the imager 146. The second and third markers 322 and 323 may be used for guiding the dilator catheter 120 through the vessel 111 and determining when the dilator catheter 120 is at and/or past the lesion 112 using vascular imaging. This is useful in determining the state of compression of the lesion 112 by the tapered portion 123. Further, when the dilator catheter 120 is moveable relative to the laser catheter 230, the second and third markers 322 and 323 enable the user to view the relative positions between the dilator catheter 120 and the distal end of the laser catheter 230 using vascular imaging. Various embodiments may include any combination of one or more of the first, second and third markers 321, 322 and 323, without departing from the scope of the present teachings.

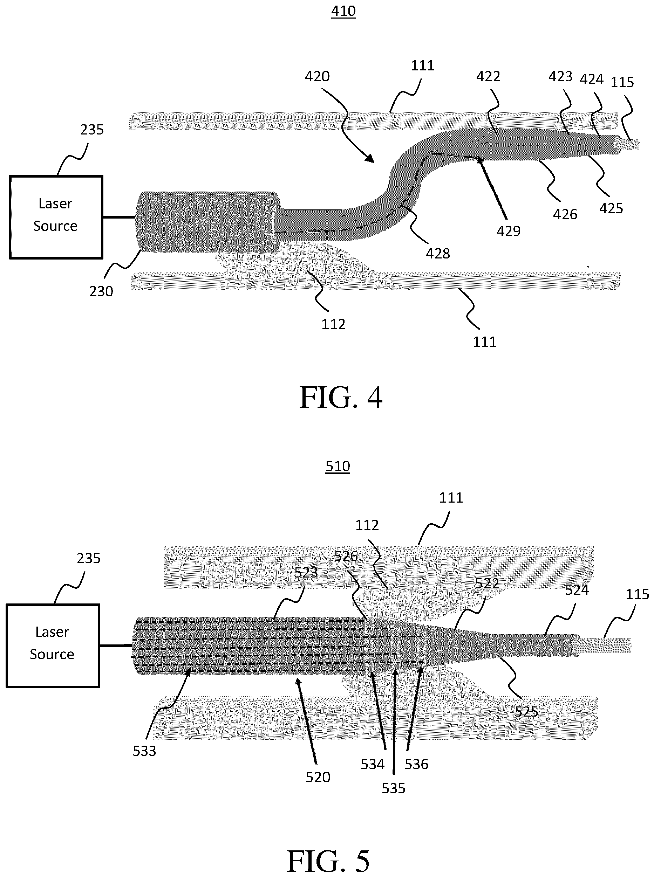

In various embodiments, the dilator catheter 120 may be steerable to provide additional maneuverability within the vessel 111. FIG. 4 is a simplified plan view of a catheter system including a laser catheter for ablating a lesion in a vessel of a subject and a steerable dilator catheter, according to a representative embodiment.

Referring to FIG. 4, catheter system 410 includes a dilator catheter 420 and the laser catheter 230 configured for insertion in the vessel 111 along the guide wire 115. The dilator catheter 420 passes through the laser catheter lumen 231 formed longitudinally in the laser catheter 230. **In** the depicted embodiment, the dilator catheter 420 is steerable using one or more pull-wires, indicated by representative pull-wire 428 (indicated by a dashed line) disposed within a pull-wire lumen in the dilator catheter 420 and connected at attachment point 429. The dilator catheter 420 is tapered as discussed above with regard to the dilator catheter 120, and therefore includes a main portion 422, a tapered portion 423 formed at the distal end of the main portion 421, and an end portion 424 formed at the distal end of the tapered portion 423. As discussed above with regard to the tapered portion 123, the tapered portion 423 increases in diameter at a first transition location 425 at the distal end of the tapered portion 423 coinciding with the proximal end the end portion 424, to a second transition location 426 at the proximal end of the tapered portion 423 coinciding with the distal end the main portion 422.

Accordingly, the user is able to compress the lesion 112 in the vessel 111 by a combination of maneuvering the dilator catheter 420 into place adjacent the lesion 112 inside the vessel 111 using the pull-wire 428 and advancing the tapered portion 423 of the dilator catheter 420 past the lesion 112 while in contact with the same. For example, the dilator catheter 420 may be formed into an s-shape (as shown) through operation of the pull-wire 428 to force the dilator catheter 420 against the lesion 112 while the tapered portion 423 is advanced past the lesion 112. The s-shape, in particular, may be realized by the pull-wire 428 being attached to a section of the dilator catheter 420 distal to the s-shape.

In various embodiments, functionality enabled by the laser catheter may be consolidated into the dilator catheter. FIG. 5 is a simplified plan view of a catheter system for ablating a lesion in a vessel of a subject without a separate laser catheter, according to a representative embodiment.

Referring to FIG. 5, catheter system 510 includes a dilator catheter 520 configured for insertion in the vessel 111 following the guide wire 115, but no separate laser catheter. The dilator catheter 520 is tapered as discussed above with regard to the dilator catheter 120, and therefore includes a main portion 522, a tapered portion 523 formed at the distal end of the main portion 522, and an end portion 524 formed at the distal end of the tapered portion 523. As discussed above with regard to the tapered portion 123, the tapered portion 523 increases in diameter from a first transition location 525 at the distal end of the tapered portion 523 to a second transition location 526 at the proximal end of the tapered portion 523.

Since there is no laser catheter, the dilator catheter 520 houses the delivery system for the laser source 235, which includes one or more optical fibers 533 (indicated by representative dashed lines) passing through the dilator catheter 520 and emitting the laser light generated by the laser source 235 from corresponding distal fiber openings, indicated by a first set of fiber openings 534, a second set of fiber openings 535, and a third set of fiber openings 536. In the depicted embodiment, the first set of fiber openings 534 is located around a perimeter of the dilator catheter 520 at the second transition location 526, the second set of fiber openings 535 is located around a perimeter of the dilator catheter 520 between at the first transition location 525 and the second transition location 526, and the third set of fiber openings 536 is located around a perimeter of the dilator catheter 520 at the first transition location 525. The laser light ablates portions of the lesion 112 at different layers associated with the corresponding outer diameters of the tapered portion 523 at which the first, second and third sets of fiber openings 534, 535 and 536 are positioned, while the lesion 112 is compressed by the dilator catheter 520.

By including the one or more optical fibers 533 in the dilator catheter 520 (without a laser catheter), the dilator catheter 520 is easier to advance because of its smaller diameter. Also, the multiple sets of fiber openings create a larger laser surface area thereby enabling ablation of a larger volume of the lesion 112 at a single pass. Also, in alternative embodiments, the dilator catheter 520 may include any combination of one or more of the first, second and third sets of fiber openings 534, 535 and 536, instead of all three sets, without departing from the scope of the present teachings. For example, the dilator catheter 520 may include only one set of fiber openings, e.g., the first set of fiber openings 534, the second set of fiber openings 535, or the third set of fiber openings 536. Or, the dilator catheter 520 may include a combination of two sets of fiber openings, e.g., the first and second sets of fiber openings 534 and 535, the second and third sets of fiber openings 535 and 536, or the first and third sets of fiber openings 534 and 536.

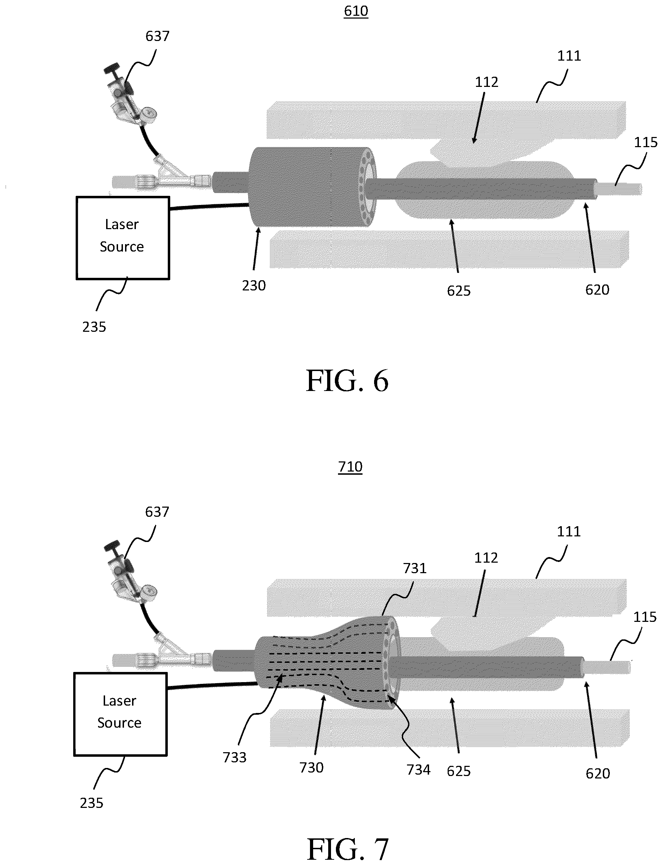

In various alternative embodiments, the tapered dilator catheter is replaced by an expandable device for compressing the lesion, such as an expandable balloon, for example. FIG. 6 is a simplified plan view of a catheter system including a laser catheter for ablating a lesion in a vessel of a subject and a dilator catheter including an expandable device, and FIG. 7 is a simplified plan view of a catheter system including an expandable laser catheter for ablating a lesion in a vessel of a subject and a dilator catheter including an expandable device, according to representative embodiments.

Referring to FIG. 6, catheter system 610 includes a dilator catheter 620 and the laser catheter 230 configured for insertion in the vessel 111 along the guide wire 115. The dilator catheter 620 passes through the laser catheter lumen 231 formed longitudinally in the laser catheter 230. In the depicted embodiment, the dilator catheter 620 includes an expandable portion 625 configured to compress the lesion 112 upon expansion. That is, the expandable portion 625 is positioned in the vessel 111 adjacent to the lesion 112 and expanded, causing it to increase from a first diameter to a second diameter that is larger than the first diameter. The increase in diameter of the expandable portion 625 in the expanded state compresses the lesion 112, which is then ablated using laser light generated by the laser source 235 and delivered by the laser catheter 230, as discussed above.

The expandable portion 625 may be a balloon, for example, that is inflated with a fluid e.g., saline and/or iodinated contrast agent. The fluid is injected into the expandable portion 625 by endoflator 637, as would be apparent to one skilled in the art. To prevent damage to the balloon, via laser light interaction with contrast agent inside the balloon, the thickness of material of the balloon may be increased, such that the laser light cannot cross the balloon material. Or, the balloon may be formed of alternative materials, such as silicone, pebax, polyester, or nylon, for example. Alternatively, or in addition, the concentration of contrast agent can be reduced via dilution with saline. In an embodiment, the balloon may be a non-compliant balloon, such that the outer diameter of the balloon is small enough for the uninflated balloon to fit into the laser catheter lumen 231 of the laser catheter 230.

FIG. 7 shows catheter system 710, which is substantially the same as the catheter system 610 except with an expandable laser catheter 730. That is, the catheter system 710 includes the dilator catheter 620 configured for insertion in the vessel 111 and the laser catheter 730 configured for insertion in the vessel 111 along the guide wire 115. The dilator catheter 620 passes through a laser catheter lumen formed longitudinally in the laser catheter 730. The dilator catheter 620 includes the expandable portion 625, such as a balloon, configured to compress the lesion 112 upon expansion, as discussed above. The laser catheter 730 includes a pliable distal end 731 configured to increase (expand) in diameter when pushed over the expandable portion 625 (e.g., inflatable balloon) when in the expanded state. Alternatively, the laser catheter 730 may be advanced over the expandable portion 625 in the non-expanded state, in which case the distal end 731 of the laser catheter 730 expands along with the expandable portion 625 when the expandable portion 625 transitions to the expanded state. The distal end 731 may be formed of a compatible flexible material, such as rubber or silicone, for example. The increase in diameter of the distal end 731 of the laser catheter 730 enables the laser light emitted by the laser source 235 to bypass the material of the expandable portion 625 when ablating the lesion 112 to avoid damage to the expandable portion 625, as discussed above.

The laser catheter 730 houses the delivery system of the laser source 235, including one or more end-emitting optical fibers 733 (indicated by representative dashed lines) passing through the laser catheter 730 and emitting the laser light generated by the laser source 235 from corresponding fiber openings 734 at the distal end 731 of the laser catheter 730. The laser light ablates a portion of the lesion 112, while the lesion 112 is compressed by the expandable portion 625. Since the laser catheter 730 is expandable, the optical fibers 733 are likewise configured to bend and deform along with the increase in diameter of the distal end 731 of the laser catheter 730. In an embodiment, the expandable laser catheter 730 may be provided, for example, as described in U.S. Patent Application Publication No. 2015/0342629 to Schneider (published December 3, 2015), which is hereby incorporated by reference in its entirety.

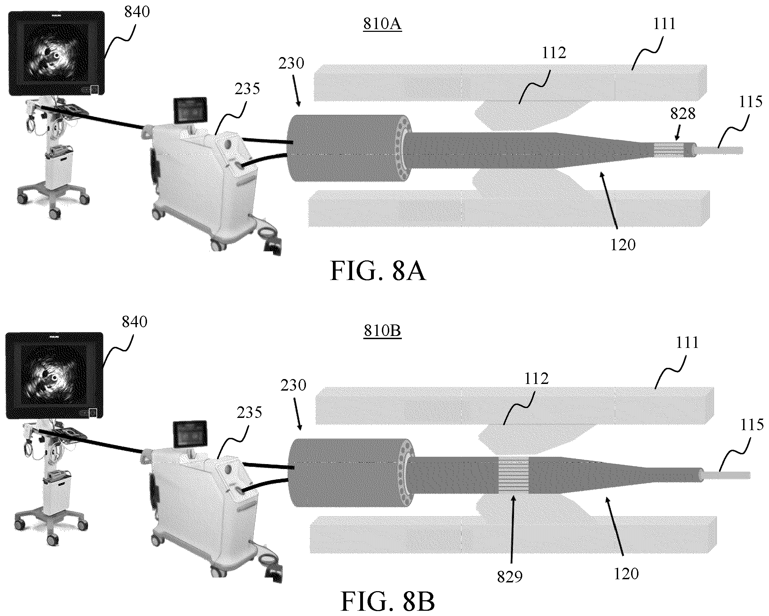

The ablation process may be further enhanced by including intravascular imaging with the catheter system, such as intravascular ultrasound (IVUS) imaging or optical coherence tomography (OCT) imaging, for example. FIG. 8A is a simplified plan view of a catheter system including a laser catheter for ablating a lesion in a vessel of a subject and a dilator catheter including a distal IVUS imaging device, and FIG. 8B is a simplified plan view of a catheter system including a laser catheter for ablating a lesion in a vessel of a subject and a dilator catheter including a middle IVUS imaging device, according to representative embodiments. Both catheter systems may include OCT imaging devices in place of the IVUS imaging devices, without departing from the scope of the present teachings.

Referring to FIG. 8A, catheter system 810A is the same as the catheter system 210 with the addition of a distal IVUS imager 828 configured to provide ultrasound images of the vessel 111 and the lesion 112 throughout the ablation procedure. That is, the catheter system 810A includes the dilator catheter 120 and the laser catheter 230 configured for insertion in the vessel 111 following the guide wire 115. In the depicted embodiment, the dilator catheter 120 is tapered as discussed above, and therefore includes a main portion 122, a tapered portion 123 formed at the distal end of the main portion 122, and an end portion 124 formed at the distal end of the tapered portion 123. As discussed above with regard to the tapered portion 123, the tapered portion 123 increases in diameter at a first transition location 125 at the distal end of the tapered portion 123 coinciding with the proximal end of the end portion 124, to a second transition location 126 at the proximal end of the tapered portion 123 coinciding with the distal end the main portion 122.

The dilator catheter 120 further includes the distal IVUS imager 828 positioned on the end portion 124 between the first transition location 125 and the distal tip of the dilator catheter 120. In the depicted embodiment, the distal IVUS imager 828 includes a set of ultrasound transducers arranged around an outer perimeter of the end portion 124. The ultrasound transducers receive electrical signals from ultrasound imaging system 840 via signal lines traversing the dilator catheter 120 (not shown), convert the electrical signals to ultrasonic waves transmitted toward the vessel walls of the vessel 111, and convert reflected ultrasonic waves back to electrical signals, which are sent to the ultrasound imaging system 840 via the signal lines. The placement of the distal IVUS imager 828 on the end portion 124 of the dilator catheter 120 enables imaging of the vessel walls and the lesion 112 as the end portion 124 is advanced past the lesion 112. Positioning the distal IVUS imager 828 on the narrower end portion 124 enables the distal IVUS imager 828 to acquire images of the lesion 112 in an uncompressed state when the end portion 124 is advanced past the lesion 112. Also, after therapy, the dilator catheter 120 may be pulled back and the distal IVUS imager 828 may be used to assess the uncompressed lesion 112 and compare it to the pre-treatment geometry.

Referring to FIG. 8B, catheter system 810B is the same as the catheter system 810A, except that the dilator catheter 120 includes a middle IVUS imager 829 positioned on the main portion 122 proximal to the second transition location 126. The middle IVUS imager 829 is configured to provide ultrasound images of the vessel 111 and the lesion 112 throughout the ablation procedure. In the depicted embodiment, the middle IVUS imager 829 includes a set of ultrasound transducers arranged around an outer perimeter of the main portion 122. Again, the ultrasound transducers receive electrical signals from ultrasound imaging system 840 via signal lines traversing the dilator catheter 120 (not shown), convert the electrical signals to ultrasonic waves transmitted toward the vessel walls of the vessel 111, and convert reflected ultrasonic waves back to electrical signals, which are sent to the ultrasound imaging system 840 via the signal lines. The placement of the middle IVUS imager 829 on the main portion 122 enables imaging of the lesion 112 itself at close proximity when the lesion 112 has been fully compressed by the main portion 122 of the dilator catheter 120. Positioning the middle IVUS imager 829 on the main portion 122 enables the middle IVUS imager 829 to monitor progression of the therapy without moving the dilator catheter 120. Also, the laser catheter 230 may be advanced and retracted over the middle IVUS imager 829 during therapy, and the reduction of the lesion 112 at that point may be visualized using the middle IVUS imager 829.

Although shown in separate embodiments, it is understood that the distal IVUS imager 828 and the middle IVUS imager 829 may be included on the same dilator catheter 120 in an alternative embodiment. Further, the distal IVUS imager 828 and/or the middle IVUS imager 829 (or OCT imagers) may be included on any of the embodiments of the dilator catheter discussed herein.

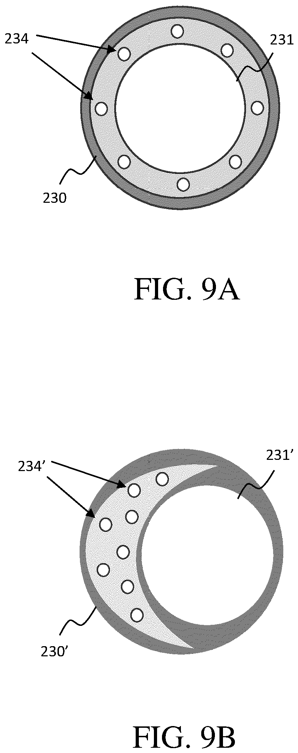

FIGs. 9A and 9B are cross-sections of the laser catheter showing symmetrical and eccentric arrangements of the corresponding laser catheter lumens and optical fiber openings, according to representative embodiments. Referring FIG. 9A, the laser catheter 230 includes the laser catheter lumen 231, which is symmetrically centered in the laser catheter 230. The dilator catheter 120 (not shown) is arranged inside the laser catheter lumen 231, as discussed above. Due to the symmetrical arrangement, the fiber openings 234 of the end-emitting optical fibers 233 passing through the laser catheter 230 are arranged concentrically around an entire perimeter of the laser catheter 230, surrounding the laser catheter lumen 231. Referring FIG. 9B, showing another embodiment, laser catheter 230' includes laser catheter lumen 231', which is positioned eccentrically (offset from center) within the laser catheter 230'. The dilator catheter 120 (not shown) is positioned within the laser catheter lumen 231'. Due to the asymmetrical arrangement, fiber openings 234' of the end-emitting optical fibers 233 passing through the laser catheter 230 are arranged within a portion of a perimeter of the distal end of the laser catheter 230', which is less than the entire perimeter of the distal end of the laser catheter 230'.

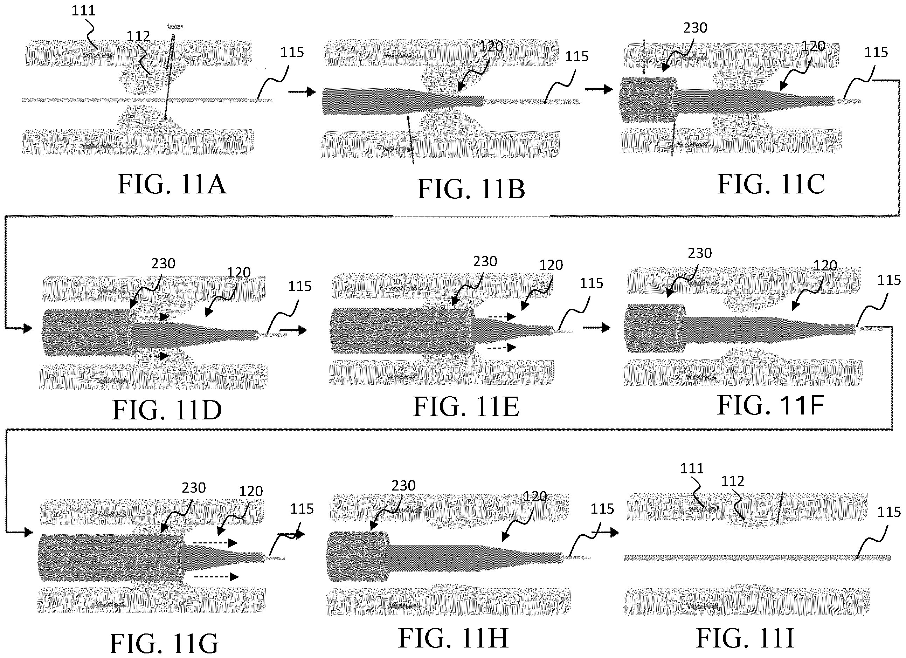

FIG. 10 is a flow diagram of a method of ablating a lesion in a vessel of a subject using a catheter system, according to a representative embodiment. For purposes of illustration, the catheter system is the catheter system 210, discussed above, which includes a dilator catheter and a laser catheter. The dilator catheter has a tapered portion that increases from a first diameter at the distal end of the tapered portion to a larger, second diameter at the proximal end of the tapered portion. The laser catheter is configured to ablate the lesion while the lesion is compressed by the tapered portion of the dilator catheter. The laser catheter includes optical fibers passing through it, where the optical fibers are exposed at a distal end of the laser catheter for emitting laser light. It is understood, however, that the general steps depicted in FIG. 10 may be implemented for other types of catheter systems and therapy devices discussed herein without departing from the scope of the present teachings. FIGs. 11A to 11I are simplified plan views a catheter system including a laser catheter for ablating a lesion in a vessel of a subject corresponding to the steps in the flow diagram of FIG. 10, according to a representative embodiment.

Referring to FIG. 10, in block S1011, the dilator catheter is inserted in the vessel of the subject and advanced through the vessel and the lesion until the tapered portion of the dilator catheter is past the lesion. The dilator catheter may be advanced along a guide wire previously inserted in the vessel, for example. As the tapered portion passes through the lesion, it causes the lesion to compress as the outer diameter of the tapered portion expands in the linear direction. FIG. 11A shows the guide wire 115 of the catheter system 210 that has been inserted in the vessel 111 past the lesion 112. FIG. 11B shows the dilator catheter 120 advancing along the guide wire 115 through the lesion 112, and FIG. 11C shows the tapered portion 123 of the dilator catheter 120 positioned past the lesion 112 from advancement of the dilator catheter 120.

In block S1012, the laser catheter is inserted in the vessel and advanced through the vessel to beginning of the lesion. The laser catheter also may be advanced along the guide wire previously inserted in the vessel, for example. Also, in an embodiment, the dilator catheter may be moveable within the laser catheter lumen relative to the laser catheter, as discussed above. In this case, the laser catheter may be advanced through the vessel to the lesion after the dilator catheter has been advanced past the lesion, although the laser catheter may be advanced at the same time as the dilator catheter during at least part of the operation, depending on the preference of the user. In another embodiment, the dilator catheter may be fixed within the laser catheter lumen relative to the laser catheter with the tapered portion of dilator catheter extending from the distal end of the laser catheter. In this case, the laser catheter is advanced through the vessel to the lesion simultaneously with the dilator catheter, although the dilator catheter will advance past the lesion prior to the laser catheter reaching the lesion due to the tapered portion of dilator catheter extending from the distal end of the laser catheter. FIG. 11C shows the laser catheter 230 advancing along the guide wire 115 over the dilator catheter 120 through the vessel 111 with the laser catheter 230 a short distance from the beginning of the lesion 112.

In block S1013, laser light is emitted from the optical fibers at a distal end of the laser catheter to perform ablation of the compressed lesion. The laser light may be emitted with the laser catheter stationary and/or while the laser catheter is being advanced past the lesion. FIG. 11D shows laser light, indicated by dashed arrows (not to scale), being emitted from the optical fibers 233 and corresponding fiber openings 234 of the laser catheter 230 as the laser catheter 230 is advanced up the lesion 112, and FIG. 11E shows the laser light being emitted from the fiber openings 234 as the laser catheter 230 is advanced past the lesion 112.

In block S1014, the laser catheter is pulled back through the lesion to the beginning of the lesion. FIG. 11F shows the laser catheter 230 on the beginning side of the lesion 112 again after being pulled back. The laser light may or may not continue to be emitted from the fiber openings 234 while the laser catheter 230 is being pulled back. As shown, the lesion 112 is smaller at this point due to the laser ablation.

In block S1015, it is determined whether the ablation is complete. In other words, it is determined whether the ablation (e.g., laser light) has reduced the size of the lesion by an amount sufficient to enable substantially unrestricted blood flow. The determination may be made by the user. For example, as discussed above, the ablation may be considered complete when the lesion is not recoiling and touching the dilator catheter when the laser catheter is retracted. That is, the ablation is complete when the laser catheter can no longer ablate lesion material because the lumen that is created in the ablated lesion is the same size or larger than the coverage of the laser catheter. This determination may be made using any compatible vascular imaging modality, such as x-ray imaging or ultrasound imaging, for example.

When it is determined that the ablation is not complete (block S1015: No), the process returns to block S1012 to repeat blocks S1012 to S1014. That is, in block S1012, the laser catheter is again advanced through the vessel to beginning of the lesion and/or rotated within the vessel. In block S1013, laser light is emitted from the optical fibers at the distal end of the laser catheter to perform additional ablation of the compressed lesion. FIG. 11G shows laser light (indicated by dashed arrows) being emitted from the optical fibers 233 and corresponding fiber openings 234 of the laser catheter 230 as the laser catheter 230 is again advanced past the lesion 112, repeating the position shown in FIG. 11E. In block S1014, the laser catheter is again pulled back through the lesion to the beginning of the lesion. FIG. 11H shows the laser catheter 230 on the beginning side of the lesion 112 after being pulled back, repeating the position shown in FIG. 11F. Notably, the lesion is shown smaller in FIG. 11H due to the repeated ablations.

Referring again to block S1015, when it is determined that the ablation is complete (block S1015: Yes), the process continues on to block S1016, according to which the laser catheter and the dilator catheter are withdrawn from the vessel. FIG. 11I shows the vessel 111 with the guide wire 115 still present following withdrawal of the laser catheter 230 and the dilator catheter 120. Remnants of the lesion 112 are shown in the vessel 111, which substantially open to unobstructed blood flow.

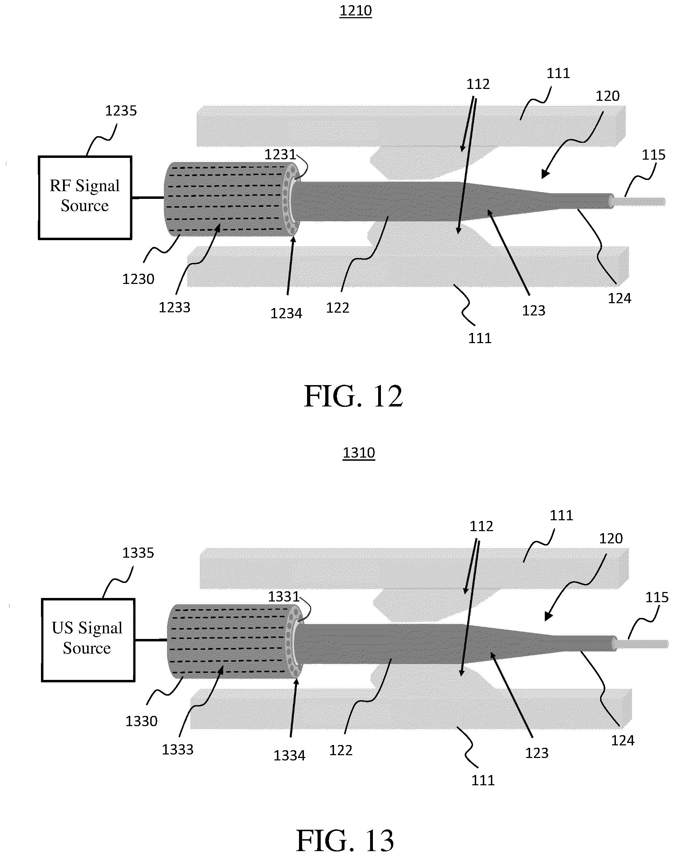

As previously mentioned, each of the embodiments discussed above with reference to a laser catheter and/or a laser source may likewise be applied to other types of therapy catheters and/or therapy devices. In this context, FIGs. 12, 13 and 14 show basic versions of the other types of therapy catheters and therapy devices that may be configured to implement each of the embodiments discussed above.

FIG. 12 is a simplified plan view a catheter system including an RF catheter for ablating a lesion in a vessel of a subject, according to a representative embodiment.

Referring to FIG. 12, catheter system 1210 is an RF catheter system shown positioned within the vessel 111 adjacent to the lesion 112 during treatment for removing the lesion 112. The catheter system 1210 includes the dilator catheter 120 and an RF catheter 1230 configured for insertion in the vessel 111 following the guide wire 115. The dilator catheter 120 passes through an RF catheter lumen 1231 formed longitudinally in the RF catheter 1230, and may be moveable or fixed therein. The therapy device is an RF signal source 1235 configured to generate RF electric current. The delivery system housed in the RF catheter 1230 includes one or more signal lines 1233 (indicated by representative dashed lines) passing through the RF catheter 1230 and corresponding electrodes 1234 configured to emit the RF electric current generated by the RF signal source 1235 from the distal end of the RF catheter 1230. The RF electric current may be generated via one of more pairs of electrodes 1234 on the RF catheter 1230 (bipolar configuration), or via one or more electrodes 1234 on the RF catheter 1230 and a patch outside the subject's body or another catheter with electrodes inside the subject's body (monopolar configuration). The RF electric current ablates a portion of the lesion 112 while the lesion 112 is compressed by the dilator catheter 120.

FIG. 13 is a simplified plan view a catheter system including a high intensity ultrasound catheter for ablating a lesion in a vessel of a subject, according to a representative embodiment.

Referring to FIG. 13, catheter system 1310 is a high intensity ultrasound catheter system shown positioned within the vessel 111 adjacent to the lesion 112 during treatment for removing the lesion 112. The catheter system 1310 includes the dilator catheter 120 and a high intensity ultrasound catheter 1330 configured for insertion in the vessel 111 following the guide wire 115. The dilator catheter 120 passes through a high intensity ultrasound catheter lumen 1331 formed longitudinally in the high intensity ultrasound catheter 1330, and may be moveable or fixed therein. The therapy device is a high intensity ultrasound signal source 1335 configured to output electrical signals for controlling the high intensity ultrasound emissions. The delivery system housed in the high intensity ultrasound catheter 1330 includes signal lines 1333 (indicated by representative dashed lines), each of which includes two leads, passing through the high intensity ultrasound catheter 1330 and corresponding ultrasound transducers 1334 configured to emit high intensity ultrasonic waves in response to the electrical signals from the distal end of the high intensity ultrasound catheter 1330. The high intensity ultrasonic waves ablate a portion of the lesion 112 while the lesion 112 is compressed by the dilator catheter 120.

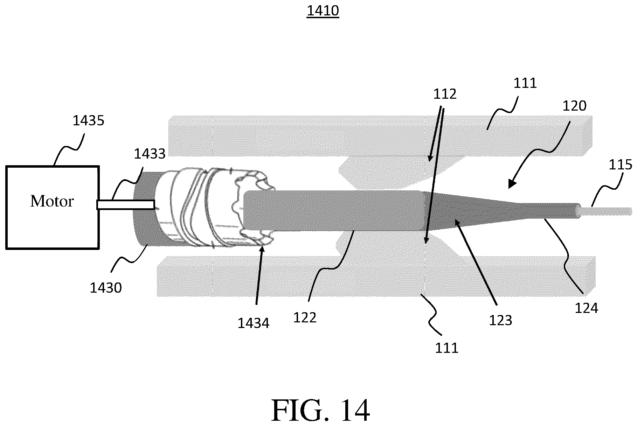

FIG. 14 is a simplified plan view a catheter system including a mechanical catheter for mechanically debulking a lesion in a vessel of a subject, according to a representative embodiment.

Referring to FIG. 14, catheter system 1410 is a mechanical catheter system shown positioned within the vessel 111 adjacent to the lesion 112 during treatment for removing the lesion 112. The catheter system 1410 includes the dilator catheter 120 and a mechanical catheter 1430 configured for insertion in the vessel 111 following the guide wire 115. The dilator catheter 120 passes through a mechanical catheter lumen 1431 formed longitudinally in the mechanical catheter 1430, and may be moveable or fixed therein. The therapy device is a motor 1435 configured to drive the delivery system housed in the mechanical catheter 1430. The delivery system includes a drive train 1433 passing through the mechanical catheter 1430 and a rotating cutter 1434 connected to the distal end of the drive train 1433 and configured cut a portion of the lesion 112 with rotating blades at the distal end of the mechanical catheter 1430.

In accordance with various embodiments of the present disclosure, the methods described herein may be implemented using a hardware computer system that executes software programs stored on non-transitory storage mediums. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

Although catheter systems for treating lesions have been described with reference to exemplary embodiments, it is understood that the words that have been used are words of description and illustration, rather than words of limitation. Changes may be made within the purview of the appended claims, as presently stated and as amended, without departing from the scope and spirit of the embodiments. Although catheter systems for treating lesions have been described with reference to particular means, materials and embodiments, it is not intended to be limited to the particulars disclosed; rather catheter systems for treating lesions extends to all functionally equivalent structures, methods, and uses such as are within the scope of the appended claims.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the Figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The Abstract of the Disclosure is provided to comply with 37 C.F.R. §1.72(b) and is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed embodiments require more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true spirit and scope of the present disclosure. Thus, to the maximum extent allowed by law, the scope of the present disclosure is to be determined by the broadest permissible interpretation of the following claims and their equivalents and shall not be restricted or limited by the foregoing detailed description.

### Further embodiments

Embodiment 1. A catheter system (110) for treating a lesion (112) in a vessel (111) of a subject, the catheter system comprising:
   a dilator catheter (120) configured for insertion in the vessel, the dilator catheter having a main portion (122) and a tapered portion (123) configured to compress the lesion as the dilator catheter is advanced through the vessel, wherein the tapered portion increases from a first diameter at a distal end of the tapered portion to a second diameter at a transition between the tapered portion and the main portion of the dilator catheter, wherein the second diameter is larger than the first diameter; and
   a therapy catheter (130) configured for insertion in the vessel, wherein the therapy catheter houses at least a portion of a delivery system (133) for a therapy device (135) configured to remove a portion of the lesion while the lesion is compressed by the dilator catheter, wherein the dilator catheter passes through a therapy catheter lumen in the therapy catheter.
Embodiment 2. The catheter system of Embodiment 1, wherein the therapy catheter comprises a laser catheter, the therapy device comprises a laser source configured to generate laser light, and the delivery system comprises at least one optical fiber passing through the laser catheter for emitting the laser light from a distal end of the laser catheter to ablate the portion of the lesion.
Embodiment 3. The catheter system of Embodiment 1, wherein the therapy catheter comprises a high intensity ultrasound catheter, the therapy device comprises a ultrasound signal source configured to control emission of high intensity ultrasound, and the delivery system comprises signal lines passing through the high intensity ultrasound catheter and at least one ultrasound transducer connected to the signal lines for emitting the high intensity ultrasound from the distal end of the high intensity ultrasound catheter to ablate the portion of the lesion.
Embodiment 4. The catheter system of Embodiment 1, wherein the therapy catheter comprises radio frequency (RF) catheter, the therapy device comprises an RF signal source configured to generate RF electric current, and the delivery system comprises at least one signal line passing through the RF catheter, and at least one electrode connected to the at least one signal line for emitting the RF electric current from the distal end of the RF catheter to ablate the portion of the lesion.
Embodiment 5. The catheter system of Embodiment 1, wherein the therapy catheter comprises a mechanical catheter, the therapy device comprises a rotating cutter, and the delivery system comprises a motor, a drive train passing through the mechanical catheter and the rotating cutter connected to the drive train at the distal end of the mechanical catheter to mechanically debulk the portion of the lesion.
Embodiment 6. The catheter system of Embodiment 1, wherein the therapy catheter comprises a first marker arranged at the distal end of the therapy catheter for guiding the therapy catheter through the vessel using a vascular imaging modality, and
   wherein the dilator catheter comprises a second marker arranged at the distal end of the dilator catheter and a third marker section arranged at a transition location of the dilator catheter for guiding the dilator catheter through the vessel and/or for determining relative positions of the dilator catheter and the therapy catheter using the vascular imaging modality.
Embodiment 7. The catheter system of Embodiment 1, wherein the dilator catheter is moveable within the therapy catheter lumen relative to the therapy catheter.
Embodiment 8. The catheter system of Embodiment 7, wherein the dilator catheter comprises a plurality of telescoping catheters moveable within the therapy catheter.
Embodiment 9. The catheter system of Embodiment 1, wherein the dilator catheter is fixed within the therapy catheter lumen relative to the therapy catheter.
Embodiment 10. The catheter system of Embodiment 1, wherein the dilator catheter has at least one pre-shaped curvature and/or is configured to be shaped into at least one curvature using at least one pull-wire.
Embodiment 11. The catheter system of Embodiment 2, wherein the at least one optical fiber passes through the dilator catheter and emits the laser light from a transition perimeter of the dilator catheter collocated with a transition location of the dilator catheter, and/or from and at least one taper perimeter of the dilator catheter located on the tapered portion distally to the transition perimeter.
Embodiment 12. The catheter system of Embodiment 1, further comprising:
   an intravascular imaging device arranged on the dilator catheter and configured to acquire intravascular images of the lesion and/or vessel walls of the vessel when the dilator catheter is inserted in the vessel.
Embodiment 13. The catheter system of Embodiment 2, wherein the therapy catheter lumen in the laser catheter containing the dilator catheter is centered in the laser catheter, and the at least one optical fiber comprises a plurality of optical fibers arranged around a perimeter of the distal end of the laser catheter.
Embodiment 14. The catheter system of Embodiment 2, wherein the therapy catheter lumen containing the dilator catheter is positioned eccentrically with the laser catheter, and the at least one optical fiber is arranged within a portion of a perimeter of the distal end of the laser catheter.
Embodiment 15. The catheter system of Embodiment 1, wherein:
   an outer diameter of the therapy catheter is about 2mm to about 7mm,
   the second diameter of the tapered portion of the dilator catheter is about 1mm to about 6mm, and
   the first diameter of the tapered portion of the dilator catheter is about 0.4mm to about 1mm.

## Claims

1. A catheter system (110) for treating a lesion (112) in a vessel (111) of a subject, the catheter system comprising:
a dilator catheter (120) configured for insertion in the vessel, the dilator catheter having a main portion (122) and a tapered portion (123) configured to compress the lesion as the dilator catheter is advanced through the vessel, wherein the tapered portion increases from a first diameter at a distal end of the tapered portion to a second diameter at a transition between the tapered portion and the main portion of the dilator catheter, wherein the second diameter is larger than the first diameter; and
a therapy catheter (130) configured for insertion in the vessel, wherein the therapy catheter houses at least a portion of a delivery system (133) for a therapy device (135) configured to remove a portion of the lesion while the lesion is compressed by the dilator catheter, wherein the dilator catheter passes through a therapy catheter lumen in the therapy catheter.

2. The catheter system of claim 1, wherein the therapy catheter comprises a laser catheter, the therapy device comprises a laser source configured to generate laser light, and the delivery system comprises at least one optical fiber passing through the laser catheter for emitting the laser light from a distal end of the laser catheter to ablate the portion of the lesion.

3. The catheter system of claim 1, wherein the therapy catheter comprises a high intensity ultrasound catheter, the therapy device comprises a ultrasound signal source configured to control emission of high intensity ultrasound, and the delivery system comprises signal lines passing through the high intensity ultrasound catheter and at least one ultrasound transducer connected to the signal lines for emitting the high intensity ultrasound from the distal end of the high intensity ultrasound catheter to ablate the portion of the lesion.

4. The catheter system of claim 1, wherein the therapy catheter comprises radio frequency (RF) catheter, the therapy device comprises an RF signal source configured to generate RF electric current, and the delivery system comprises at least one signal line passing through the RF catheter, and at least one electrode connected to the at least one signal line for emitting the RF electric current from the distal end of the RF catheter to ablate the portion of the lesion.

5. The catheter system of claim 1, wherein the therapy catheter comprises a mechanical catheter, the therapy device comprises a rotating cutter, and the delivery system comprises a motor, a drive train passing through the mechanical catheter and the rotating cutter connected to the drive train at the distal end of the mechanical catheter to mechanically debulk the portion of the lesion.

6. The catheter system of any one of claims 1 to 5, wherein the therapy catheter comprises a first marker arranged at the distal end of the therapy catheter for guiding the therapy catheter through the vessel using a vascular imaging modality, and
wherein the dilator catheter comprises a second marker arranged at the distal end of the dilator catheter and a third marker section arranged at a transition location of the dilator catheter for guiding the dilator catheter through the vessel and/or for determining relative positions of the dilator catheter and the therapy catheter using the vascular imaging modality.

7. The catheter system of any one of claims 1 to 6, wherein the dilator catheter is moveable within the therapy catheter lumen relative to the therapy catheter.

8. The catheter system of claim 7, wherein the dilator catheter comprises a plurality of telescoping catheters moveable within the therapy catheter.

9. The catheter system of any one of claims 1 to 6, wherein the dilator catheter is fixed within the therapy catheter lumen relative to the therapy catheter.

10. The catheter system of any one of claims 1 to 9, wherein the dilator catheter has at least one pre-shaped curvature and/or is configured to be shaped into at least one curvature using at least one pull-wire.

11. The catheter system of claim 2, wherein the at least one optical fiber passes through the dilator catheter and emits the laser light from a transition perimeter of the dilator catheter collocated with a transition location of the dilator catheter, and/or from and at least one taper perimeter of the dilator catheter located on the tapered portion distally to the transition perimeter.

12. The catheter system of any one of claims 1 to 11, further comprising:
an intravascular imaging device arranged on the dilator catheter and configured to acquire intravascular images of the lesion and/or vessel walls of the vessel when the dilator catheter is inserted in the vessel.

13. The catheter system of claim 2, wherein the therapy catheter lumen in the laser catheter containing the dilator catheter is centered in the laser catheter, and the at least one optical fiber comprises a plurality of optical fibers arranged around a perimeter of the distal end of the laser catheter.

14. The catheter system of claim 2, wherein the therapy catheter lumen containing the dilator catheter is positioned eccentrically with the laser catheter, and the at least one optical fiber is arranged within a portion of a perimeter of the distal end of the laser catheter.

15. The catheter system of any one of claims 1 to 14, wherein:
an outer diameter of the therapy catheter is about 2mm to about 7mm,
the second diameter of the tapered portion of the dilator catheter is about 1mm to about 6mm, and
the first diameter of the tapered portion of the dilator catheter is about 0.4mm to about 1mm.
